# EUROPÄISCHE PATENTSCHRIFT

(11) **EP 0 332 892 B1**
(45) Veröffentlichungstag und Bekanntmachung des Hinweises auf die Patenterteilung: **18.05.1994**
(21) Anmeldenummer: 89103054.6
(22) Anmeldetag: 22.02.1989
(51) Int. Cl.: A61B 5/103, A61C 19/04

(54) **Vorrichtung zur Bestimmung der relativen Bewegung zweier Messstellen zueinander**
Device for determining the relative movement of two measuring positions
Dispositif pour déterminer le mouvement relatif de deux emplacements de mesure

(30) Priorität: 26.02.1988 DE 3806029
(43) Veröffentlichungstag der Anmeldung: 20.09.1989
(73) Patentinhaber: RAP - RECHNERGESTÜTZTE AUTOMATISATIONS- UND PRÜFTECHNIK RISS GMBH, D-38106 Braunschweig (DE)
(72) Erfinder: Schrader, Erhard, Dr., D-3330 Helmstedt (DE); Riss, Wolfgang, Dipl.-Ing., D-3320 Salzgitter 1 (DE); Mende, Gottfried, Dr. Ing., D-3302 Cremlingen (DE)
(74) Vertreter: Lins, Edgar, Dipl.-Phys. Dr.jur.

(56) Entgegenhaltungen:
- EP-A- 0 025 201
- DE-A- 3 312 245
- US-A- 4 234 306
- JOURNAL OF THE ACOUSTICAL SOCIETY OF AMERICA, Band 72, Nr. 3, September 1982, Seiten 700-704, Acoustical Society of America, New York, US; Y. SONODA et al.: "New optical method for recording lip and jaw movements"

## Beschreibung

Für die verschiedensten Anwendungsfälle sind Vorrichtungen bekannt, mit denen die relative Bewegung zweier Meßstellen zueinander bestimmt werden kann. Die Verformung von Trägern bzw. Stäben unter einer Last läßt sich beispielsweise mit Hilfe einer Fernsehkamera erkennen. Durch die Fernsehkamera wird die Vorrichtung relativ teuer. Ihrer Verwendbarkeit sind darüber hinaus Grenzen gesetzt. So ist es erforderlich, daß der Träger oder Stab bei der Belastung sichtbar ist. Problematisch ist die Anwendung darüber hinaus, wenn eine Verformung auf kleinstem Raum festgestellt werden soll. Problematisch ist ferner eine Langzeitüberwachung, beispielsweise die Bestimmung eines Kriechvorganges einer Klebung unter Belastung.

Im zahnmedizinischen Bereich ist es für die Herstellung von Zahnersatz wesentlich, diesen so zu gestalten, daß die natürliche Artikulationsbewegung des Unterkiefers relativ zum Oberkiefer beibehalten wird und sich die Zähne gegenseitig in geeigneter Weise gegeneinander abstützen. Hierzu wird die individuelle Artikulationsbewegung registriert und in einem geeigneten Artikulator simuliert, in den ein Modell des Patientengebisses eingesetzt wird. Zur Herstellung des Zahnersatzes wird daher immer wieder auf die simulierte Artikulationsbewegung Bezug genommen.

Zur Registrierung der Artikulationsbewegung wird eine Meßvorrichtung benutzt, die am Unterkiefer befestigt wird und einen zu den Unterkiefergelenken (Kondylen) ragenden Bügel aufweist. Der sogenannte Kondylenpol wird dabei abgetastet und eine Schreibspitze des Gesichtsbogens auf diesen Punkt ausgerichtet. Unter die Schreibspitze wird ein Registrierungsblatt gelegt, auf das während der Kaubewegung des Patienten die Bewegung der Kondylen aufgezeichnet wird.

Das übereinstimmend ausgeführte Meßprinzip besteht darin, die Bewegung des Unterkiefers festzustellen, um die individuelle Bewegung des Unterkiefers zum Oberkiefer bei der Herstellung des Zahnersatzes im zahntechnischen Labor möglichst genau zu simulieren. Hierzu sind weiterentwickelte Geräte bekanntgeworden, z. B. der Stereo-Gnathograph-V der Dental-Elektronik KG in 8480 Weiden. Das mechanische Aufzeichnungssystem ist dabei durch ein berührungsloses Bewegungserfassungssystem ersetzt worden, das im Ohrbereich drei Infrarotsender und drei Infrarotempfänger aufweist, zwischen denen eine in geeigneter Weise ausgebildete Platte bewegbar ist, die mit dem Gesichtsbogen verbunden ist. Die Bewegung am kondylenseitigen Ende des Gesichtsbogens wird durch das berührungslose Bewegungserfassungssystem erfaßt und kann elektronisch aufgezeichnet werden. Am eigentlichen Meßprinzip ist hierbei jedoch nichts geändert.

Durch die EP-A-0 025 201 ist eine Vorrichtung bekannt, die zwei Platten aufweist, die in den Oberkiefer und in den Unterkiefer eingesetzt werden. Die in den Oberkiefer eingesetzte Platte ist mit einem Sender versehen, während die in den Unterkiefer eingesetzte Platte drei Empfänger aufweist, die ein etwa gleichseitiges Dreieck bilden. Der Sender bildet mit dem Empfänger eine Abstandsmeßeinrichtung, wobei die Laufzeit der Sendesignale vom Sender zum jeweiligen Empfänger bestimmt wird.

Durch DE-A-33 12 245 ist ferner ein Gerät zur Diagnose von Unterkieferbewegungen bekannt, das ein fest mit dem Kopf verbundenes - und somit fest zum Oberkiefer angeordnetes - Teil und ein zusammen mit dem Unterkiefer bewegbares Teil aufweist. An dem unteren Teil sind Lichtquellen angeordnet, für die am oberen Teil Doppelspalte und dahinter liegende lineare Sensoren vorgesehen sind. Das diesem Gerät zugrundeliegende Meßprinzip besteht darin, die Bewegung dreier, untereinander in einer festen geometrischen Anordnung verbundener Punkte durch Koordinaten jeweils in einer Beobachtungsebene zu beschreiben. Durch die Doppelspalte entstehen Lichtlinien, die auf dem linearen Sensor bezüglich Ihres Abstandes zu einem Nullpunkt detektierbar sind. Die Anordnung setzt daher für jede Lichtquelle eine eigene Sensoranordnung voraus, mit der lineare Koordinaten feststellbar sind. Eine derartige Anordnung ist umständlich zu montieren, erlaubt keinen Einsatz in räumlich beengten Verhältnissen und ist schwierig zu handhaben.

Der Erfindung liegt die Aufgabe zugrunde, eine Vorrichtung der eingangs erwähnten Art so auszubilden, daß die Bestimmung der relativen Bewegung auf kleinstem Raum mit einer relativ einfachen Vorrichtung möglich ist.

Zur Lösung dieser Aufgabe dient erfindungsgemäß eine Vorrichtung zur Bestimmung der relativen Bewegung zweier Meßstellen zueinander mit
- zwei Halterungen, die an den beiden Meßstellen angebracht sind,
- einer auf einer Halterung befestigten Senderanordnung,
- einer auf einer Halterung befestigten Empfängeranordnung zur Detektion von Auftreffpunkten der von der Senderanordnung emittierten Strahlen,
- wobei
   - die Senderanordnung wenigstens drei Strahlenquellen aufweist, von denen wenigstens zwei zueinander schräg stehen und
   - die Halterung der Empfängeranordnung eine Platte mit einer Sensorfläche ist.

Die Erfindung beruht auf der Erkenntnis, daß eine komplette Bestimmung der Bewegung zweier Meßstellen zueinander mit Hilfe einer derartigen Senderanordnung und einer Empfängeranordnung möglich ist, wenn die Empfängeranordnung die Auftreffpunkte der zwischen den beiden Halterungen verlaufenden Strahlen detektiert. Durch eine geeignete Anordnung der Strahlenquellen können Abstandsänderungen sowie relative Kippbewegungen der beiden Halterungen zueinander mit der Empfängeranordnung erkannt werden. Die Strahlenquellen der Senderanordnung können eigenständige Strahlengeneratoren sein oder auch von einem gemeinsamen Strahlengenerator gespeist werden, beispielsweise durch Lichtleiter gebildet sein, die von einer gemeinsamen Lichtquelle, beispielsweise eine Laserdiode, versorgt werden. Die erfindungsgemäße Vorrichtung ist selbstverständlich nicht auf die Anwendung von Lichtstrahlen beschränkt. Es können sowohl elektromagnetische Strahlen in einem anderen Frequenzbereich als auch nicht elektromagnetische Strahlenanordnungen, beispielsweise Ultraschallwellen o. ä. verwendet werden.

Eine sehr zweckmäßige Senderanordnung besteht aus wenigstens zwei parallel zueinander stehenden Strahlenquellen und wenigstens einer dazu schräg stehenden Strahlenquelle. Mit einer derartigen Anordnung lassen sich sowohl Entfernungsänderungen als auch Parallelverschiebungen und Kippbewegungen, zumindest in einer Richtung, zuverlässig detektieren. Vorzugsweise stehen die parallel zueinander stehenden Strahlenquellen in der Ausgangslage der beiden Platten im wesentlichen senkrecht zu der gegenüberliegenden Platte.
Die Erfindung soll im folgenden anhand von in der Zeichnung näher dargestellten Ausführungsbeispiele näher erläutert werden. Es zeigen:
- Figur 1 -: einen Prinzipaufbau für drei Lichtquellen auf einer Senderplatte relativ zu einem Sensor auf einer Empfängerplatte sowie eine prinzipielle Darstellung verschiedener Bewegungen der Platten zueinander
- Figur 2 -: eine Darstellung eines Oberkiefers und eines Unterkiefers mit einer eingesetzten Senderplatte und einer eingesetzten Empfängerplatte
- Figur 3 -: eine Anordnung mit Sender- und Empfängerplatte an einem Träger
- Figur 4 -: eine Anordnung mit einer Sender- und Empfängerplatte an einer Klebstelle
- Figur 5 -: eine Anordnung mit Sender- und Empfängerplatte an einem Parallellenkeraufbau.

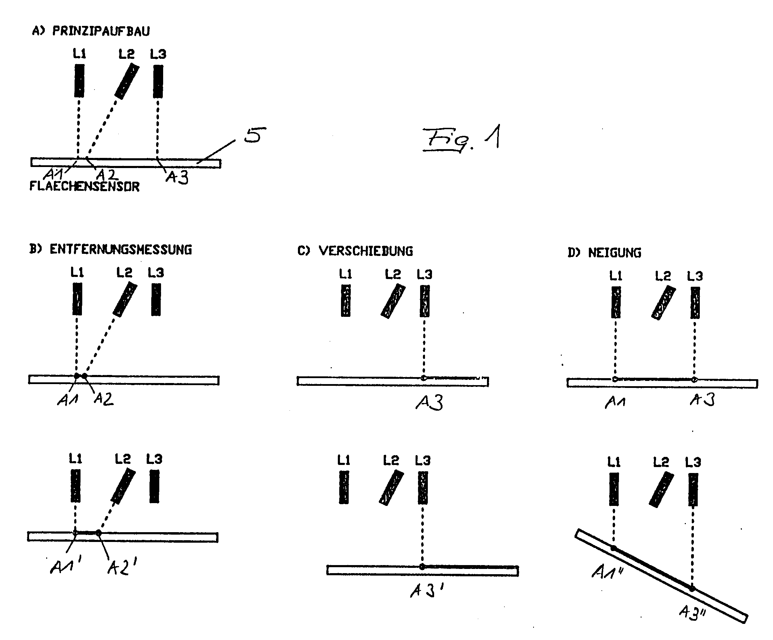

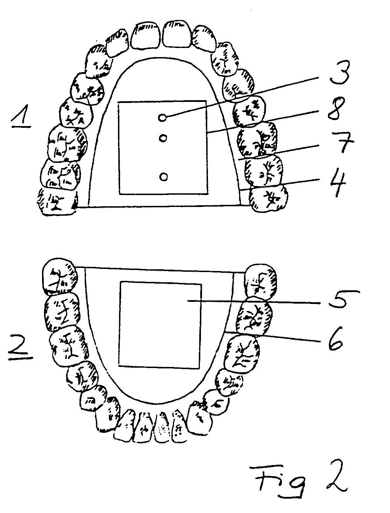

Figur 1 zeigt in der Abbildung A eine Prinzipanordnung für drei Strahlenquellen 3, die hier als Lichtquellen L1, L2, L3 ausgebildet sind. Diese drei Lichtquellen müssen keine selbständige Lichterzeuger sein, sondern können beispielsweise durch Lichtleiter gebildet sein, denen von einem gemeinsamen Lichtgenerator Lichtsignale zugeleitet werden. In einfacher Weise können aber auch normale Glühlampen, Leuchtdioden oder vorzugsweise Laserdioden als Lichtquellen L1, L2, L3 eingesetzt werden.

Die drei auf der Senderplatte 4 befestigten Lichtquellen L1, L2, L3 sind so angeordnet, daß zwei der Lichtquellen, nämlich L1 und L3 parallel zueinander stehen, während eine mittlere, auf der Verbindungslinie der beiden Lichtquellen L1, L3 angeordnete Lichtquelle L2 schräg dazu abstrahlt.

Die drei Lichtquellen erzeugen auf der Sensorfläche 5 Auftreffpunkte A1, A2, A3, deren Lage durch die Sensorfläche 5 detektiert wird.

Wie Abbildung B der Figur 1 zeigt, wird für eine Entfernungsmessung nur das Licht der Lichtquellen L1, L2 benötigt. Wandert die Sensorfläche 5, und damit der Unterkiefer 2 näher an die Senderplatte 4, und damit an den Oberkiefer 1 heran, vergrößert sich der Abstand zwischen den Auftreffpunkten A1′, A2′ der Lichtquellen L1, L2, wenn sich die Strahlen der Lichtquellen L1, L2 in der Ausgangsstellung nicht geschnitten haben.

In Abbildung C ist dargestellt, daß für die Feststellung einer Verschiebung der Sensorfläche 5 nur ein Auftreffpunkt benötigt wird. Die Lage beispielsweise des Auftreffpunktes A3 auf der Sensorfläche 5 ändert sich, wenn diese parallel zu sich selbst verschoben wird, wie dies durch den Auftreffpunkt A3′ verdeutlicht ist.

Abbildung D zeigt die Detektion einer Neigung der beiden Platten 4, 6 zueinander. Die beiden parallel zueinander stehenden Lichtquellen L1, L3 erzeugen bei einer parallelen Ausrichtung der beiden Platten 4, 6 zueinander Auftreffpunkte A1, A3, deren Abstand dem Abstand der Lichtquellen L1, L3 entspricht. Wird die Sensorfläche 5 relativ zu der Senderplatte 4 geneigt, entstehen Auftreffpunkte A1'', A3'', deren Abstand voneinander sich proportional zur Neigung in Richtung der Verbindungslinie der beiden Lichtquellen L1, L3 vergrößert. Selbstverständlich ist für die relative Neigungsbestimmung nicht erforderlich, daß die beiden Platten 4, 6 in der Ausgangslage völlig parallel zueinander stehen.

Die in Figur 1 dargestellte Anordnung dient zur Detektion der relativen Bewegungen zweier Meßstellen M1 und M2 entlang einer Detektionslinie. Eine dreidimensionale Detektion der Bewegung ist durch entsprechende Ergänzung von Lichtqellen, beispielsweise senkrecht zu der Verbindungslinie der Lichtquellen L1, L2, L3 ohne weiteres möglich.

Die Auswertung der ermittelten Meßwerte während eines Bewegungsvorganges wird von einem Rechner vorgenommen. Die Wiedergabe der ermittelten Bewegung ist ohne weiteres in zwei- oder drei-dimensionaler Form möglich.

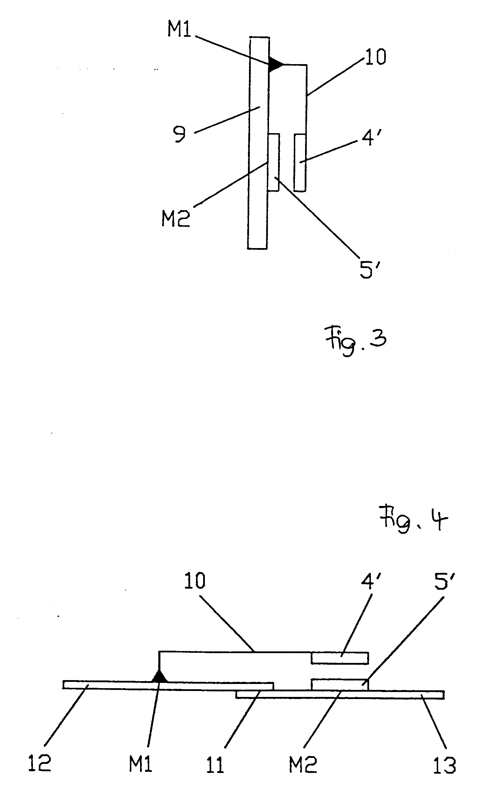

Figur 2 zeigt ein Anwendungsbeispiel im zahnmedizinischen Bereich. Zur Analyse der Artikulationsbewegung von Oberkiefer 1 zu Unterkiefer 2 ist in den Oberkiefer 1 eine Strahlenquellen 3 tragende erste Platte 4 eingesetzt, die als Senderplatte fungiert, während in den Unterkiefer 2 eine eine Sensorfläche 5 tragende Platte 6 eingesetzt ist. Beide Platten 4, 6 sind mit geeignetem Ausgleichsmaterial 7 eingepaßt.

Die erste Platte 4 (Senderplatte) hält eine Trägerplatte 8, die drei als Strahlenquellen fungierende Lichtquellen 3 trägt.

Die im Unterkiefer eingesetzte zweite Platte 6 (Empfängerplatte) trägt eine Sensorfläche 5, die die Detektion von Auftreffpunkten der Lichtstrahlen mit den Koordinaten auf der Sensorfläche 5 ermöglicht. Hierzu wird die Sensorfläche 5 zeilenförmig abgetastet, wobei jedem Auftreffpunkt ein definierter elektrischer Wert zugeordnet ist.

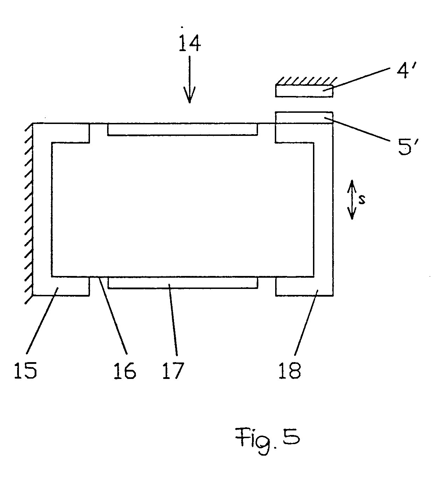

Figur 3 zeigt die Anwendung von Senderplatte 4′ und Empfängerplatte 5′ zur Emittlung der Verformung eines Trägers 9 unter Belastung. Die Senderplatte 4′ ist dabei mit einem Befestigungswinkel 10 an einer Meßstelle M1 befestigt, während die Empfängerplatte 5′ unmittelbar an einer zweiten Meßstelle M2 angebracht ist. Aufgrund des Befestigungswinkels 10 liegen Senderplatte 4′ und Empfängerplatte 5′ einander gegenüber. Eine Verformung des Trägers zwischen Meßstelle M1 und Meßstelle M2 wird mit Hilfe des in Figur 1 beschriebenen Prinzipaufbaus detektiert.

Figur 4 zeigt ein Ausführungsbeispiel für eine Klebstelle 11, die zwei Teilstücke 12, 13 miteinander verbindet. Mit der in Figur 3 dargestellten Anordnung aus Senderplatte 4′, Empfängerplatte 5′ und Befestigungswinkel 10 läßt sich die relative Bewegung der Meßstelle M1 auf dem ersten Teilstück 12 zu der Meßstelle M2 auf dem zweiten Teilstück 13 unter Belastung der Klebzone 11 bestimmen.

In dem in Figur 5 dargestellten Ausführungsbeispiel wird mit Hilfe einer Senderplatte 4, und einer Empfängerplatte 5′ die Bewegung eines Parallellenkeraufbaus 14 analysiert. An einer ortsfesten Halterung 15 sind über zwei Filmgelenke 16 zwei parallele Lenker 17 angelenkt, an deren Ende ein parallel geführter Aufbau 18 eine Bewegung in Richtung des Doppelpfeils S ausführen soll. Zur Analyse dieser Bewegung ist der Aufbau 18 mit der Empfängerplatte 5′ versehen, der gegenüber der Senderplatte 4′ ortsfest befestigt ist.

Es ist ohne weiteres ersichtlich, daß die dargestellte Ausführungsbeispiele nicht zu limitieren sind und beliebig ergänzt werden können. Die erfindungsgemäße Vorrichtung eignet sich für die Bestimmung der relativen Bewegung zweier Meßstellen M1, M2 zueinander insbesondere, wenn nur ein geringer Raum zur Verfügung steht. Die erfindungsgemäße Vorrichtung kann in Maschinen o. ä. an von außen nicht sichtbaren Stellen eingebaut sein, so daß eine Bewegungsanalyse auch dann möglich ist, wenn auf Sichtkontakt beruhende Vorrichtungen versagen.

## Patentansprüche

1. Vorrichtung zur Bestimmung der relativen Bewegung zweier Meßstellen (M1, M2) zueinander mit
- zwei Halterungen (4, 5), die an den beiden Meßstellen (M1, M2) angebracht sind,
- einer auf einer Halterung (4, 5) befestigten Senderanordnung,
- einer auf einer Halterung (4, 5) befestigten Empfängeranordnung zur Detektion von Auftreffpunkten der von der Senderanordnung emittierten Strahlen,
- wobei
- die Senderanordnung wenigstens drei Strahlenquellen (L1, L2, L3) aufweist, von denen wenigstens zwei zueinander schräg stehen und
- die Halterung der Empfängeranordnung (6) eine Platte (5) mit einer Sensorfläche ist.

2. Vorrichtung nach Anspruch 1, bei der auch die Halterung der Senderanordnung eine Platte (4) ist.

3. Vorrichtung nach Anspruch 2, dadurch gekennzeichnet, daß die eine Platte (4) die Senderanordnung und die andere Platte (5) die Empfängeranordnung aufweist.

4. Vorrichtung nach einem der Ansprüche 1 bis 3, dadurch gekennzeichnet, daß die Senderanordnung wenigstens eine dritte Strahlenquelle (L3) aufweist, die parallel zu einer anderen Strahlenquelle (L1) steht.

5. Vorrichtung nach Anspruch 3 und 4, dadurch gekennzeichnet, daß die parallel zueinanderstehenden Strahlenquellen (L1, L3) in der Ausgangslage der beiden Platten (4, 5) im wesentlichen senkrecht zu der Empfängerplatte (5) stehen.

## Claims

1. Apparatus for determining the relative movement of two measurement points (M1, M2) with respect to one another, having
- two mountings (4, 5) which are set up at the two measurement points (M1, M2),
- a transmitter arrangement secured to a mounting (4, 5),
- a receiver arrangement secured to a mounting (4, 5), for detecting impact points of the beams emitted by the transmitter arrangement,
- the transmitter arrangement having at least three beam sources (L1, L2, L3), of which at least two are oblique with respect to one another, and
- the mounting of the receiver arrangement (6) being a plate (5) having a sensor surface.

2. Apparatus according to Claim 1, in which the mounting of the transmitter arrangement is also a plate (4).

3. Apparatus according to Claim 2, characterized in that the one plate (4) has the transmitter arrangement and the other plate (5) has the receiver arrangement.

4. Apparatus according to one of Claims 1 to 3, characterized in that the transmitter arrangement has at least one third beam source (L3) which is parallel to another beam source (L1).

5. Apparatus according to Claim 3 and 4, characterized in that the mutually parallel beam sources (L1, L3) are, in the starting position of the two plates (4, 5), substantially perpendicular to the receiver plate (5).

## Revendications

1. Dispositif pour déterminer le mouvement relatif de deux points de mesure (M1, M2) l'un par rapport à l'autre, comprenant :
- deux montures (4, 5), qui sont rapportées aux deux points de mesure (M1, M2),
- un agencement émetteur fixé sur une monture (4, 5),
- un agencement récepteur fixé sur une monture (4, 5) pour détecter des points d'impact des rayons émis par l'agencement émetteur,
dans lequel
- l'agencement émetteur comporte au moins trois sources de rayonnement (L1, L2, L3) dont deux au moins sont orientées obliquement l'une par rapport à l'autre et
- la monture de l'agencement récepteur (6) est une plaque (5) comportant une surface sensible.

2. Dispositif selon la revendication 1, dans lequel la monture de l'agencement émetteur est également une plaque (4).

3. Dispositif selon la revendication 2, caractérisé en ce que l'une des plaques (4) présente l'agencement émetteur et l'autre plaque (5) l'agencement récepteur.

4. Dispositif selon l'une des revendications 1 à 3, caractérisé en ce que l'agencement émetteur présente au moins une troisième source de rayonnement (L3), qui est orientée parallèlement à une autre source de rayonnement (L1).

5. Dispositif selon la revendication 3 et 4, caractérisé en ce que dans la position de départ des deux plaques (4, 5) les sources de rayonnement (L1, L3) orientées parallèlement l'une à l'autre sont orientées sensiblement perpendiculairement à la plaque réceptrice (5).
